# EUROPEAN PATENT APPLICATION

(11) **EP 4 597 162 A1**
(43) Date of publication of application: **06.08.2025**
(21) Application number: 24315035.6
(22) Date of filing: 30.01.2024
(51) Int. Cl.: G01S 7/52, A61B 8/08

(54) **A METHOD OF OPTIMIZING AN ULTRASOUND IMAGE**

(71) Applicant: SUPERSONIC IMAGINE, 13290 Aix-en-Provence (FR)
(72) Inventor: Marsac, Laurent, 13090 Aix-en-Provence (FR)
(74) Representative: Paustian & Partner Patentanwälte mbB

(57) **Abstract**

The invention relates to a method of optimizing an ultrasound image. The method comprises:

• selecting a first ultrasound image generated by using a first beamforming method,
• accessing spatio-temporal signal data associated with the first ultrasound image, and
• beamforming the spatio-temporal signal data using a second beamforming method,

wherein the first beamforming method is different from and computationally less expensive than the second beamforming method.

## Description

### BACKGROUND

Ultrasound imaging provides a non-invasive method to visualize the internal structure of a medium, for example a material, an animal, a human body or parts of it (for example organs). In particular, visualization methods can be used to screen for and detect a lesion in a medium (for example a tissue of a patient).

The aim of ultrasound imaging is to estimate the medium reflectivity. In a conventional ultrasound imaging method, an ultrasound transducer device (also referred to as an ultrasound probe) with a set of ultrasound transducer elements may be used. In the method, one or multiple transducers are used to transmit one or successively several ultrasound beam into a medium, corresponding to a transmission operation. Then, in a reception operation a set of backscattered echo signals are received from the medium by the set of transducer elements. In particular, each of the transducer elements converts a received echo signal into for example an electrical signal. The signal may further be processed by the ultrasound system. For example, they may be amplified, filtered digitalized and/or a signal conditioning operation may be carried out. The transducer elements may be arranged as a transducer array.

Said signals may then be transmitted to an image processing system. The received signals may be processed to generate image data of the scanned medium, for example using a beamforming method. Generally, beamforming may be understood as a signal processing technique conventionally used in sensor arrays for directional signal transmission or reception. This process is used to generate beamformed data. In other words, beamforming may be understood as a signal processing technique that is achieved by combining elements in an antenna array (for example an ultrasound transducer) in such a way that the combined signals form constructive interferences.

Conventionally, a user of an ultrasound system can perform an ultrasound scan using the ultrasound probe and simultaneously watch the processed ultrasound images on a display device of the system in real-time or quasi real-time. Such a real-timed or quasi real-time feedback of the system helps the user to control the ultrasound scan, i.e. to focus with the probe on the region of interest in the medium. For example, after having started a scan, the user can stop the current scan (for example by pressing a "freeze" button" of the system) and subsequently review the scanned and processed image sequence in a so-called "cine loop".

However, if for example a potential abnormality (for example a lesion) is found in an image, it can be desirable for the user to verify and to investigate the abnormality in more detail. Conventionally, the user can perform a further ultrasound scan to obtain more images of the medium. However, if the quality of the images is insufficient, it may not be helpful to obtain more images of the same weak quality or the patient may not be available anymore.

In view of the above, there is a need to solve such drawbacks and associated disadvantages.

### SUMMARY OF THE DISCLOSURE

A method of optimizing an ultrasound image is provided. The method comprises:
selecting a first ultrasound image,
accessing spatio-temporal signal data (for example stored) associated with the first ultrasound image, and
beamforming the spatio-temporal signal data using a second beamforming method.

By providing such a method, a first ultrasound image can be provided and thus made selectable, for example by using a first (for example conventional) beamforming method which may be relatively fast and/or computationally inexpensive. In other words, the first ultrasound image may be provided in real-time or quasi real-time to the user.

The term "quasi real-time" may mean that a delay caused by computation is not and almost not perceivable by a user of system performing the method of the present disclosure, for example with a delay of 0.01s or less.

For example, the first image may be generated and displayed to a user during an ultrasound scan. The user may perform an ultrasound scan using an ultrasound probe and simultaneously watch the processed first ultrasound images on a display device of or linked to the system. This allows the user to better control the scan in real-time or quasi real-time, for example to focus using the probe on a region of interest in the scanned medium. Also a sequence of first ultrasound image may be generated during the ultrasound scan.

As described in more detail below, the second beamforming method may be an enhanced and/or computationally more expensive beamforming method compared to a first (for example conventional) beamforming method which may be used for generating the first ultrasound image based on the spatio-temporal signal data.

The accessed spatio-temporal signal data may be stored spatio-temporal signal data. In other words, the spatio-temporal signal data may not be kept "on the fly" during generation of the first ultrasound image and then be lost as in a volatile data organization for example. Instead, the spatio-temporal signal data may be for example collected (for example using an ultrasound probe) and stored (for example in a memory device, in particular in a FIFO dedicated memory).

Accordingly, it is possible to (re-)beamform the spatio-temporal signal data using the second beamforming method, in order to obtain an ultrasound image with an increased quality. In this context, it is not problematic, if the required computational effort (in view of the performance of the used processing unit) does not allow an image generation in real-time or quasi real-time. The user may anyway reliably control the ultrasound scan using the first ultrasound image (or sequence of images).

After the ultrasound scan has been completed, the user may select a first ultrasound image which for example advantageously shows a region of interest in the scanned medium. For this purpose the user may for example review a sequence of first images in a so-called "cine loop", as described above. If required by the user, a second ultrasound image may then be obtained which corresponds to the first ultrasound image but of an increased quality, after a certain calculation delay of for example a 0.1s or up to a few seconds.

An increased image quality may mean at least one of: an increased contrast and/or resolution, a reduced noise, sharper contours of regions of interest, etc. In , general the increased image quality which is made possible by using the second beamforming method may allow a user to more reliably analyze a region of interest, for example to detect and/or localize an abnormality, such as a lesion based on a highest quality image available thanks to the disclosure.

The spatio-temporal signal data may be ultrasound data.

The spatio-temporal signal data may be or may comprise pre-beamformed spatio-temporal signal data and/or radiofrequency (RF) data.

The spatio-temporal signal data comprises a plurality of oscillating and/or periodic signals received from the medium and/or sections of the plurality of oscillating signals
Performing an ultrasound scan may mean collecting the spatio-temporal signal data using at least an ultrasound probe.

The method may be a computer-implemented method. Computer may be associated or not with the probe.

A second optimized image may be generated by at least one of: beamforming the spatio-temporal signal data, and/or by using the second beamforming method.

The first ultrasound image may be generated using a first (for example conventional) beamforming method which may be different to the (for example enhanced or optimized) second beamforming method. Hence, the first and second beamforming methods may process the same spatio-temporal signal data and thus generate images of the same region in the medium, but with different qualities and also different zoom levels or selected region(s).

The first beamforming method may be computationally less expensive than the second beamforming method. For example, the first beamforming method may allow to obtain the first ultrasound image during an ultrasound scan in real-time or quasi real-time, as explained above.

The first and the second ultrasound image may be generated using the same ultrasound system.

The first and the second beamforming method may be performed by the same ultrasound system.

The method may further comprise (in particular before and/or during beamforming the spatio-temporal signal data using the second beamforming method): determining a parameter of the medium as a function of image data of the first ultrasound image and/or the spatio-temporal signal data. For example, a location of a ROI (region of interest) in the medium may be used to adjust a focal area in the second beamforming method.

The second beamforming method may be adjusted as a function of the determined parameter of the medium.

The parameter may comprise at least one of:
an attenuation of sound of the medium,
a speed of sound of the medium,
a location of a region of interest in the medium
a visco-elasticity of the medium.

For example, such a parameter may be a characteristic representative of the medium.

The parameter of the medium may be determined as a function of a signal parameter of the spatio-temporal signal data.

It is also possible that the parameter is the signal parameter. The signal parameter of the spatio-temporal signal data may be derived from the spatio-temporal signal data.

In still other words, the parameter of the medium may be determined as a function of the spatio-temporal signal data by determining a signal parameter of the spatio-temporal signal data

Accordingly, it may be distinguished between different types of parameters, i.e., first, characteristics of the medium itself and, second, characteristics of the spatio-temporal signal data. Of course, also a characteristic of the medium may be at least indirectly derived from the spatio-temporal signal data, for example derived from a signal parameter.

The signal parameter may comprise at least one of:
a frequency,
an amplitude,
a focal depth,
a frame rate,
phase property.

Examples of a phase property may comprise at least one among:
· signs,
· phases,
· a sign proportion,
· phase intervals,
· phase evolution states, and proportions of phase properties.

Before beamforming the spatio-temporal signal data using a second beamforming method, the method may comprise filtering the spatio-temporal signal data using a filter, wherein the filter may be adjusted as a function of the determined parameter of the medium.

For example, the filter may be configured to filter a predetermined spectrum of the spatio-temporal signal data. According to a particular example, a certain frequency spectrum may be determined to be noisy. This spectrum may be filtered out by the respectively adjusted filter(s).

The second beamforming method may comprise at least one of:
a retrospective transmit beamforming (RTB) method,
an adaptative beamforming method,
a matrix beamforming method.

An example of a matrix beamforming method is described in patent application filed by and referenced FR3114159.

An example of a retrospective transmit beamforming (RTB) method is described in patent application referenced US20220287685A1.

An adaptive beamforming method may for example use artificial intelligence (AI) algorithms to adapt the beamforming process.

Examples of AI models may comprise machine learning models, such as decision trees, random forests, logistic regression, gradient boosting, neural networks, and/or support vector machines. Predefined algorithms may be used to perform classification and prediction tasks, such as image recognition. For example, a decision tree algorithm may be used to classify images of objects based on their features. Other examples of machine leaning models may comprise deep learning models, such as neural networks, k-nearest neighbors, and/or clustering algorithms, which may be used for tasks such as anomaly detection and data segmentation. AI may refer to the development of computer systems and algorithms that may perform tasks that typically require human intelligence, such as visual perception, speech recognition, decision-making, and language translation. AI systems and algorithms may be trained using làrge sets of data and sophisticated algorithms that may allow them to identify patterns and trends, make predictions, and optimize outcomes.

The method may further comprise, before selecting a first ultrasound image:
collecting and storing a first set of spatio-temporal signal data over a first time period,
generating (for example simultaneously and/or in real-time or quasi real-time) a first sequence of first ultrasound images by beamforming associated spatio-temporal signal data of the first set,
optionally stopping collecting and storing the first set (i.e. by "freezing" the last image of the second sequence) and thus stopping the first sequence of first images, wherein
for each first image of the first sequence: storing the first image and assigning the first image to the associated spatio-temporal signal data of the first set.

The method may further comprise:
storing the spatio-temporal signal data of the first set and the associated first images of the first sequence in a FIFO (First In First Out) memory,
displaying at least one stored first image, and
selecting the displayed first image.

For example, the memory may be part of the ultrasound system used to perform the method of the present disclosure, or external to said system. The FIFO memory may be configured to keep only the most recent spatio-temporal signal data and to successively replace (i.e. delete) older data by newly collected ones. In this way, the user can always access the most recent data, and at the same time the memory can have a limited size.

The method may further comprise accessing the stored spatio-temporal signal data associated with the selected first ultrasound image, and beamforming the spatio-temporal signal data using the second beamforming method.

The method may further comprise, after generating a first sequence of first ultrasound images or optionally after generating a second optimized image:
collecting and storing a second set of spatio-temporal signal data over a second time period (for example being after the first time period),
generating (for example simultaneously and/or in real-time or quasi real-time) a second sequence of first ultrasound images by beamforming associated spatio-temporal signal data of the second set,
optionally stopping collecting and storing the second set (i.e. by "freezing" the last image of the second sequence) and thus stopping the second sequence of first images, wherein
for each first image of the second sequence: storing the first image and assigning the first image to the associated spatio-temporal signal data of the second set.

Accordingly, after generating a first image sequence (i.e. a first "cine loop"), the user may review the images and optionally generate at least one second optimize image. In case none of these images (i.e. of the first image sequence and/or the optional second image) is satisfying, the user may generate a second image sequence (i.e. a second "cine loop"). Hence, the method in one embodiment allows an interactive procedure where the user can obtain additional spatio-temporal signal data (i.e. the second set), based on the usability of the previously obtained spatio-temporal signal data (i.e. the first set).

The first and/or second image sequence may be each a so-called "cine loop" which can be reviewed by a user.

The first and the second ultrasound image show at least partially the same region of a medium.

The spatio-temporal signal data may be associated with ultrasound waves insonified into a medium.

The present disclosure may further refer to a computing device, comprising: at least one processor, and
at least one memory storing computer-executable instructions, the computer-executable instructions when executed by the processor cause the computing device to perform a method according to any one of the preceding methods aspects.

For example, the computing device may be configured to receive a selected first ultrasound image (or related selection information), access (for example stored) spatio-temporal signal data associated with the first ultrasound image, and beamform the spatio-temporal signal data using a second beamforming method.

The processor (or processing unit) may be a component of electronic devices that may be responsible for carrying out computational tasks. There may be different types of processing units, each designed for specific purposes. A processing unit may be or may comprise a Central Processing Unit (CPU), Graphics Processing Unit (GPU), Digital Signal Processor (DSP), Field-Programmable Gate Array (FPGA), and/or Application-Specific Integrated Circuit (ASIC). The CPU may be the primary processing unit in a computer that may be responsible for executing most of the instructions and calculations required by the computer. GPUs may be specialized processing units that may be designed to handle the complex calculations required for rendering graphics and video. DSPs may be specialized processing units that may handle signal processing tasks, while FPGAs may be reconfigurable processing units that may be programmed to perform various computational tasks. ASICs may be customized processing units designed to perform a specific set of tasks, optionally making them highly efficient and effective for their intended purpose. These processing units may be found in a wide range of electronic devices, such as medical electronic devices. Medical electronic devices may include computers, smartphones, and other digital devices, optionally enabling these devices to perform various computational tasks efficiently and accurately. The method according to the present disclosure may also run on a virtual server and/or a distant server.

The present disclosure may also relate to an ultrasound system for optimizing an ultrasound image, the system comprising means for carrying out the method according to any examples of the present disclosure. For example, the system may comprise or may be a computing device, as described above.

The present disclosure may also relate to a computer program comprising instructions which, when the program is executed by a computer, cause the computer to carry out the method according to any examples of the present disclosure.

In case the method operations may comprise any aspects (for example physical) which go beyond a mere data processing (for example an ultrasound signal processing), the computer program may further comprise computer-readable instructions which when executed by a data processing system cause any external elements of a system (for example an ultrasound transducer device) to carry out these operations.

The present disclosure may also relate to a computer-readable medium comprising instructions which, when executed by a computer, cause the computer to carry out the method according to any examples of the present disclosure.

It is intended that combinations of the above-described elements and those within the specification may be made, except where otherwise contradictory.

It is to be understood that both the foregoing general description and the following detailed description are exemplary and explanatory only, are provided for illustration purposes and are not restrictive of the disclosure, as claimed.

The accompanying drawings, which are incorporated in and constitute a part of this specification, illustrate examples of the disclosure and together with the description, and serve to support and illustrate the principles thereof.

### BRIEF DESCRIPTION OF THE DRAWINGS

Fig. 1 schematically shows a method of optimizing an ultrasound image according to an example of the present disclosure.
Fig. 2 schematically shows a pipeline for optimizing an ultrasound image according to examples of the present disclosure.
Fig. 3 shows a schematic drawing of an ultrasound system 10 according to examples of the present disclosure.

### DESCRIPTION OF THE DRAWINGS

Reference will now be made in detail to examples of the disclosure, examples of which are illustrated in the accompanying drawings. Wherever possible, the same reference numbers will be used throughout the drawings to refer to the same or like parts.

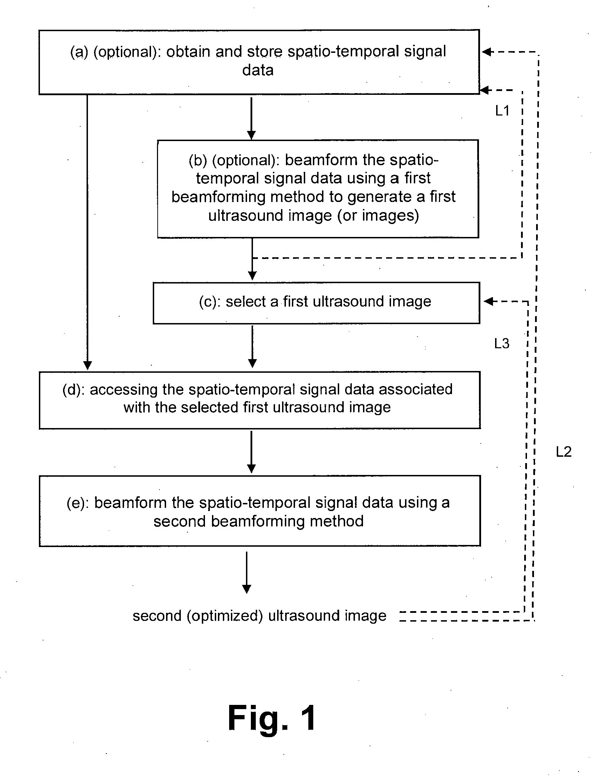

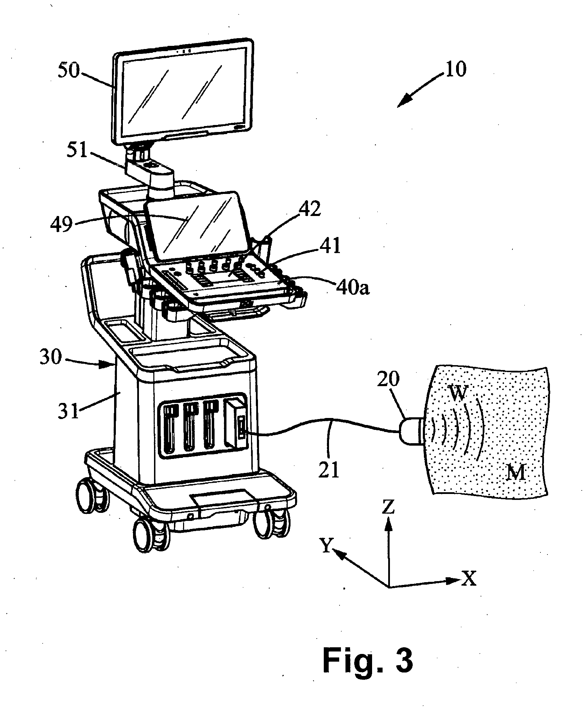

Fig. 1 schematically shows a method of optimizing an ultrasound image according to an example of the present disclosure. The method may be implemented in the system of Fig. 3.

The method may comprise an optional operation (a) of obtaining and storing spatio-temporal signal data. The spatio-temporal signal data may be associated with a medium. In other words, the data may comprise information of the medium.

The spatio-temporal signal data may be obtained by transmitting an ultrasound pulse into the medium and by receiving and collecting in response spatio-temporal ultrasound signals from the medium.

More in detail, operation (a) may comprise transmitting a pulse into the medium. For example, the transmission operation may comprise insonification of the medium with a cylindrical wave that focuses on a given point and/or plane waves of different angles. More in particular, during the transmission operation, a plurality of ultrasonic waves may be transmitted into a spatial region.

Generally, in the present disclosure a pulse may correspond to an acoustic signal emitted by a transducer element. The pulse may for example be defined by at least one of: the pulse duration, the frequency of the resulting wave, the number of cycles at the given frequency, the polarity of the pulse, etc. A wave may correspond to the wavefront generated by one or several transducer elements (i.e. by respectively emitted pulses). The wave may be controlled by means of emission delay between the different used transducer elements. Examples comprise a plane wave, a focused wave and a divergent wave. A beam may correspond to the physical area insonified by the wave (for example in the medium). Hence, the beam may be related to the wave but may have less or no temporal notion. For example, it may be referred to a beam when the depth of field of a focused beam is of interest.

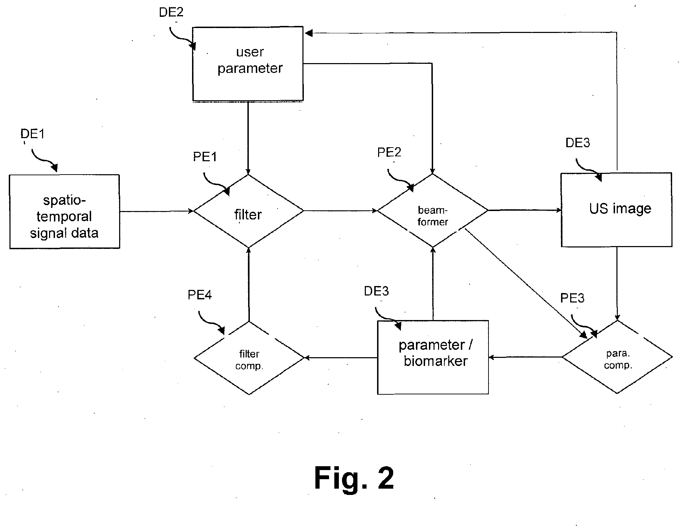

In response, a plurality of signals may be received from the medium by the plurality of transducer elements. The plurality of signals may comprise backscattered echoes of the transmission of transmission operation. The response sequence may also be referred to as spatio-temporal data and/or signal data, in particular ultrasound signal data and/or RF and/or IQ signal data. The signal data may be in the time domain, more in particular in a spatio-temporal domain, as for example described in more detail below. In one example and as described in more detail in context of fig. 2, the response sequence may be processed by bandpass filtering, in order to keep only one or several frequency ranges.

The received signals may be digitalized, in order to obtain digital spatio-temporal signal data.

The spatio-temporal signal data may be stored in a memory device of the ultrasound system or an external system.

In an optional operation (b) the spatio-temporal signal data may be beamformed using a first beamforming method to generate a first,ultrasound image (or images).

The first beamforming method may be relatively fast and/or computationally inexpensive. In other words, the first ultrasound image may be provided in real-time or quasi real-time to the user.

For example, the first ultrasound image may be generated and displayed to a user during an ultrasound scan. The user of a respective of an ultrasound system may perform an ultrasound scan using an ultrasound probe and simultaneously watch the processed first ultrasound images on a display device of the system. This allows the user to better control the scan in real-time or quasi real-time, for example to focus using the probe on a region of interest in the scanned medium. As described in more detail below, a sequence of first ultrasound image may be generated during the ultrasound scan.

For example, the first ultrasound image may comprise a B-mode (brightness mode) image and/or ShearWave^{™} Elastography image or of any other ultrasound modality.

In operation (c) a first ultrasound image may be selected. For example, one or several generated, i.e. beamformed, images may be displayed on a display device to a user. A user may select one or several first ultrasound images, for example using a user interface of the ultrasound system.

In one example, after the ultrasound scan has been completed (cf. operation (a) and (b)), the user may select a first ultrasound image which for example advantageously shows a region of interest in the scanned medium. For this purpose the user may for example review a sequence of first images in a so-called "cine loop".

In operation (d) the spatio-temporal signal data associated with the selected first ultrasound image may be accessed. Accordingly, the spatio-temporal signal data obtained in operation (a) may at least be stored until they are accessed in operation (d).

For example, in operation (d) based on a look-up table, a database or therelike, a subset of spatio-temporal signal data may be identified which is associated with, i.e. is related to the selected first image. Then, this subset may be accessed and for example transferred to a processing unit (for example a graphical processing unit, GPU) for further processing in operation (e). Correspondingly, for a plurality of selected images a respective plurality of subsets may be accessed.

In operation (e) the spatio-temporal signal data associated with the selected image may be beamformed using a second beamforming method. As a result, a second optimized ultrasound image may be generated by operation (e).

The second beamforming method may be an enhanced and/or computationally more expensive beamforming method compared to the first (for example conventional) beamforming method which may be used for generating the first ultrasound image based on the spatio-temporal signal data.

For example, the second beamforming method may comprise at least one of a retrospective transmit beamforming (RTB) method, an adaptative beamforming method, and a matrix beamforming method.

Accordingly, by using an optimized and/or more enhanced second beamforming method, a second ultrasound image of an advantageously increased image quality may be obtained implying for example: an increased contrast and/or resolution, a reduced noise, sharper contours of regions of interest, etc. In general the increased image quality which is made possible by using the second beamforming method may allow a user to more reliably analyze a region of interest, for example to detect and/or localize an abnormality, such as a lesion.

Operations (a) and (b) may be repeated in a first loop L1. In each iteration of loop L1; a (further) first ultrasound image may be generated. Hence, a sequence of first ultrasound images may be generated, as long as spatio-temporal signal data are obtained (for example, as long as a user scans a medium with an ultrasound probe).

The first ultrasound images of this sequence and the associated spatio-temporal signal data may be available in operation (c) for a selection. It is also possible that only the latest first images and associated data are available, as far as they are (still) available in a FIFO memory.

It is also possible that several image sequences may be generated, in case for example user does not find any available image of a given image sequence as suitable for a selection.

Operations (a) and (e) may be repeated in a second loop L2. For example, a user may review a second optimized ultrasound image and wish to obtain more information from the medium. In this case, the procedure may be restarted in operation (a).

In still another option, operations (c) and (e) may be repeated in a second loop L2. For example, a user may review a second optimized ultrasound image and wish to obtain more optimized second image information based on the available first images. In this case, the user may select another first image in operation (c) or may select the same first image in operation (c) but with different parameters (cf. explanations in context of fig. 2).

It is noted that all operations (a) to (e) may be performed in one embodiment by the same ultrasound imaging system. Hence, even though this system may have limited capacities, e.g. in terms of its (relatively) compact size, its computation performance etc. a user can imagine the medium in real-time or quasi real-time (due to the generated first ultrasound images) and anyway high-quality ultrasound images (i.e. the second ultrasound images) can be provided.

It is however possible, that at least some operations are performed by other/different devices. For example, the operations of transmitting a pulse into the medium and receiving the signal in response may also be carried out by any other system than the system used for operations (b) to (e). Moreover, it is also possible that operations (d) and (e) are performed by another system that operation (b). Operations (a) and (b) are thus only optional.

Fig. 2 schematically shows a pipeline for optimizing an ultrasound image according to examples of the present disclosure.

The pipeline may consist of different process modules PEx which handle (i.e. process) several data elements DEx. The process modules may be software modules. Accordingly, the complete pipeline may be a software implemented pipeline. In other words, the pipeline may be implemented as now or several computer programs. The pipeline (i.e. the respective software program) may run on the ultrasound system, as for example described in context of fig. 3, or at least partially on an external system.

Furthermore, the pipeline may be configured to perform the method of fig. 1 and optionally have further functions or capabilities.

The process modules may be operated simultaneously or successively, depending on their need. For example, each process module may be available at any time and may process input data, when needed.

The data element DE1 may consist of spatio-temporal signal data, which may be obtained by an ultrasound probe and stored in memory device.

The spatio-temporal signal data may be fed into an optional process module PE1 which is configured to filter the spatio-temporal signal data.

For example, the filter may be adjusted by user parameters DE2 which may be manually set by a user.

Moreover, the filter may be automatically adjusted as a function of filter parameters computed by a process module PE4.

For example, the filter may be configured to filter a predetermined spectrum of the spatio-temporal signal data. According to a particular example, a certain frequency spectrum may be determined to be noisy by process module .PE4 based on a determined parameter DE3 (as explained in more detail below). This spectrum may be filtered out by the respectively adjusted filter PE1.

Hence, the user parameters DE2 may allow a user to manually re-adjust any automatically determined filter parameters.

The optionally filtered spatio-temporal signal data may be fed into a process module PE2 which is configured to beamform the spatio-temporal signal data. The process module PE2 may be configured to perform any kind of beamforming method, at least the first and second beamforming method according to the present disclosure. Accordingly, an ultrasound image or a sequence of images DE3 may be generated by the process module PE2.

Again, the process module PE2 may be manually adjusted by user parameters DE2. For example, the ultrasound image(s) DE3 may be provided to the data element DE2. Accordingly, a user parameter may include the selection of one or several images DE3, based on which the second beamforming method is performed according to the present disclosure by the process element PE2.

Moreover, the beamforming method performed by process module PE2 may be automatically adjusted as a function of determined parameters DE3 (as explained in more detail below).

The process module PE2 may generate an ultrasound (US) image, i.e. depending on the user beamforming' method, a first or second ultrasound image according to the present disclosure (or a plurality of images, for example a sequence of first ultrasound images).

An optional process module PE3 may be configured to compute or determine a parameter DE3 based on the ultrasound image(s). Accordingly, process module PE3 may determine parameter of the medium as a function of beamformed image data and optionally of the spatio-temporal signal data.

The parameter may for example be a medium parameter (or parameter of the medium), which represents a particular characteristic of the medium. Examples of a medium parameter may include an attenuation of sound of the medium, a speed of sound of the medium, a location of a region of interest in the medium (for example of a potential lesion), or a visco-elasticity of the medium.

The parameter may also be a signal parameter, which represents a particular characteristic of the medium of the spatio-temporal signal data.

Accordingly, the parameter of the medium may be derived from or correspond to a signal parameter which determined as a function of the spatio-temporal signal data (filtered or not filtered).

It may thus be distinguished between different types of parameters, i.e., first, characteristics of the medium itself and, second, characteristics of the spatio-temporal signal data. Of course, also a parameter of the medium may be at least indirectly derived from the spatio-temporal signal data, for example derived from a signal parameter.

Examples of signal parameters include a frequency, an amplitude, a focal depth, a frame rate, and phase property. Particular examples of a phase property may comprise at least one among signs, phases, a sign proportion, phase intervals, phase evolution states, and proportions of phase properties.

Further examples of parameters DE2 and/or DE3 may comprise settings, such as processing parameters (for example speed of sound, edge enhancement, speckle reduction) and display parameters (for example dynamic range, quantification range) or other (for example a mode, gain, persistence, compression, harmonic imaging, spatial compounding, speckle reduction, edge enhancement, color Doppler settings, pulsed wave Doppler settings, and power Doppler settings, etc. It is noted that the afore-mentioned list is not exhaustive and may also comprise further settings to configure the filter PE1 and/or the beamforming method PE2.

In a further example, a biomarker may be derived from or determined based on one or several parameters. The biomarker may be used to adapt the process element PE2 and/or the process element PE2 (similarly to the parameter, as described above).

Generally, a biomarker may be a coefficient, based on which an anomality of the medium (for example a disease) may be determined. The biomarker may hence be used as for example a health indicator. Accordingly, the biomarker may be used to detect or confirm the presence of a disease or condition of interest, or a subtype of the disease.

For example, the biomarker may be determined based on a physical characteristic and/or a combination of several physical characteristics of a medium, which can be affected by a disease of a patient or an organ of a patient.

In one example, a biomarker may be determined based on or correspond to a speed of sound in a medium. The speed of sound measured in for instance a fatty liver can be different to the speed of sound measured in a normal liver. Accordingly, such a biomarker may be used as a health indicator of the liver.

Coming back to process element PE2, the beamforming method may be adjusted as a function of the parameter of data element DE3. For example, in a first iteration, the process element PE2 may use the first beamforming method according to the present disclosure (for example a relatively fast method), in order to generate a sequence of first ultrasound images DE3 according to the present disclosure. This beamformer may not have any individually parameter adjustment by a parameter DE3 yet.

However, based on generated image data (of image(s) DE3) a parameter DE3 may be computed. Then, in a second iteration, the process element PE2 may use the second beamforming method according to the present disclosure (for example a relatively enhanced method), in order to generate a one or several selected second ultrasound images DE3 according to the present disclosure (for example selected according to a user parameter DE2).

Said second beamforming method may be adjusted as a function of a parameter DE3.

For example, a location of a ROI (for example a potential lesion) in the medium may be determined based on the image data of the first ultrasound image(s) DE3. Said location information may be used to adjust a focal area in the second beamforming method. The focal area may be for example ameliorated by reducing noise or clutter in this area caused by other areas. For example, only the image information (i.e. pixel information) of this region of interest may be generated by the second beamforming method and optionally be laid over the first ultrasound image. The region of interest may be manually localized by a user or may be automatically localized by the process element PE3, for instance by using an AI (artificial intelligence) based recognition algorithm.

According to a further example, the process element PE3 may determine a phase property (i.e. a particular signal parameter) based on the spatio-temporal signal data (for example filtered spatio-temporal signal data). Based on the phase property the speed of sound in the medium may be determined (i.e. a particular parameter of the medium). Said parameter of the medium may be used to adjust the second beamforming method, such that the generated second ultrasound image(s) are ameliorated.

It is noted that the beamforming method of process element PE2 may be adjusted and thus ameliorated in a plurality of iterations. For example, a second ultrasound image generated by a second, i.e. enhanced beamforming method may be used to obtain a parameter PE3 which used to further adjust and thus optimize the second beamforming method. Accordingly, after said further adjustment or optimization, a new and thus further optimized second image may be generated.

Moreover, the parameter DE3 or the biomarker DE3 may be used to adjust the filter PE1 using the process element PE4 for computing the filter. For example, an attenuation coefficient (i.e. a parameter of the medium) DE3 may be taken into account by the process element PE4 to adapt the filter (for example by amplifying higher frequencies of the received response signals of more distanced / more profound layers of the medium, which may be attenuated by the medium).

The adjusted filter may be used to (re-) filter the spatio-temporal signal data DE1, and feed filtered data to the process element PE3. PE3 may use the filtered data for generating an image DE3 (for example using an adjusted second beamforming method, as described above).

Hence also the filter adjustment may be done in several iterations, each time using an optimized parameter DE3.

Due to the possibility of performing several iterations in the pipeline of fig. 2, as described above, the quality of the ultrasound image DE3 may successively be optimized. Likewise, the accuracy and/or reliability of a biomarker DE3 may successively be increased. Hence, also a disease or anomality may be determined more accurately and/or more reliably.

Fig. 3 shows a schematic drawing of an ultrasound system 10 according to examples of the present disclosure. The ultrasound system 10 may be configured to perform the method of fig. 1 and/or the pipeline of fig. 2. Accordingly, the ultrasound system 10 may be an example of a system for optimizing an ultrasound image. However, the method according to the present disclosure may also be (at least partially) performed by an external system.

The ultrasound imaging system 10 may comprise:
- a probe 20 (i.e. an ultrasound transducer device having a plurality of transducer elements),
- a processing unit 30 for processing an image on the bases of signals received by the probe,
- a control panel 40a connected to the processing unit, said control panel at least comprising buttons 41 and a touch pad 42, and
- a display 50 for visualizing the image.

The probe 20 may be associated to the processing unit 30 via a cable 21 or via a wireless connection, and it is able to emit ultrasound waves W into a medium M and to receive ultrasound waves W from the medium M, said received ultrasound waves being consequent or resulting from reflections of said emitted ultrasound waves on diffusing particles inside said medium M.

The display screen 50 may be a screen for visualizing the image processed by the processing unit 30. The display 50 may also visualize other information such as scales used in the image, or configuration information for the processing or any information such as help information or contextual gesture help for the touch pad 42.

The display screen may by articulated on a support arm 51 for better positioning for the user. The display screen is usually a high-definition screen of a great size (at least 20 inches) for better image visualization to the user.

The control panel 40a is for example a portion of the system casing 31, said portion comprising a panel casing having a substantially flat surface inclined towards the user for manipulation by one hand of said user. The control panel 40a may be moved by a hanger upwards and downward for being adapted to the user size, and may be optionally moved frontward and rearward for being adapted to the user position. The control panel 40a may include a control panel display screen 49 for visualizing several configuration information or any information dedicated to the user.

The processing unit 30 is configured to determine an ultrasound acquisition parameter according to the present disclosure. The system may further display first and/or second ultrasound images, as described in context of Fig. 1 and 2, in particular with an option to select one or several first ultrasound images, in order to generate correspond second ultrasound image(s).

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially" and/or "approximately" and/or "generally" should be understood to mean falling within such accepted tolerances.

The terms "record" and "receive" may be used synonymously throughout this disclosure unless denoted differently.

Although the present disclosure herein has been described with reference to particular examples, it is to be understood that these examples are merely illustrative of the principles and applications of the present disclosure.

It is intended that the specification and examples be considered as exemplary only, with a true scope of the disclosure being indicated by the following claims.

A reference herein to a patent document or any other matter identified as prior art, is not to be taken as an admission that the document or other matter was known or that the information it contains was part of the common general knowledge as at the priority date of any of the claims.

## Claims

1. A method of optimizing an ultrasound image, comprising:
selecting a first ultrasound image,
accessing spatio-temporal signal data associated with the first ultrasound image, and
beamforming the spatio-temporal signal data using a second beamforming method.

2. The method of claim 1, wherein
a second optimized image is generated by at least one of:
beamforming the spatio-temporal signal data, and
using the second beamforming method.

3. The method of claim 1 or 2, wherein
the first ultrasound image is generated using a first beamforming method which is different to the second beamforming method.

4. The method according to any one of the preceding claims, wherein
the first beamforming method is computationally less expensive than the second beamforming method.

5. The method according to any one of the preceding claims, wherein
the first and the second ultrasound image are generated by the same ultrasound system, and/or
the first and the second beamforming method are performed by the same ultrasound system.

6. The method according to any one of the preceding claims, further comprising:
determining a parameter of the medium as a function of image data of the first ultrasound image and/or the spatio-temporal signal data, wherein
the second beamforming method is adjusted as a function of the determined parameter of the medium.

7. The method according to the preceding claim, wherein
the parameter comprises at least one of:
an attenuation of sound of the medium,
a speed of sound of the medium,
a location of a region of interest in the medium
a visco-elasticity of the medium.

8. The method according to any one of the preceding claims 6 or 7, wherein
the parameter of the medium is determined as a function of a signal parameter of the spatio-temporal signal data.

9. The method according to the preceding claim, wherein
the signal parameter comprises at least one of:
a frequency,
an amplitude,
a focal depth,
a frame rate,
phase property.

10. The method according to any one of the preceding claims, wherein
before beamforming the spatio-temporal signal data using a second beamforming method,
filtering the spatio-temporal signal data using a filter, wherein the filter is adjusted as a function of the determined parameter of the medium.

11. The method according to any one of the preceding claims, wherein
the second beamforming method comprise at least one of:
a retrospective transmit beamforming (RTB) method,
an adaptative beamforming method,
a matrix beamforming method.

12. The method according to any one of the preceding claims, further comprising before selecting a first ultrasound image:
collecting and storing a first set of spatio-temporal signal data over a first time period,
generating a first sequence of first ultrasound images by beamforming associated spatio-temporal signal data of the first set, wherein
for each first image of the first sequence: storing the first image and assigning the first image to the associated spatio-temporal signal data of the first set.

13. The method according to the preceding claim, further comprising:
storing the spatio-temporal signal data of the first set and the associated first images of the first sequence in a FIFO memory,
displaying at least one stored first image, and
selecting the displayed first image.

14. The method according to any one of the preceding claims 12 or 13, further comprising after generating a first sequence of first ultrasound images or optionally after generating a second optimized image:
collecting and storing a second set of spatio-temporal signal data over a second time period,
generating a second sequence of first ultrasound images by beamforming associated spatio-temporal signal data of the second set,
for each first image of the second sequence: storing the first image and assigning the first image to the associated spatio-temporal signal data of the second set.

15. The method according to any one of the preceding claims, wherein
the first and the second ultrasound image show at least partially the same region of a medium.
